(19) Europäisches Patentamt
European Patent Office
Office européen des brevets

(11) Numéro de publication: **0 437 144 B1**

(12) **FASCICULE DE BREVET EUROPEEN**

(45) Date de publication de fascicule du brevet: **30.11.94**

(51) Int. Cl.[5]: **C07C 237/46**, A61K 49/04

(21) Numéro de dépôt: **90403770.2**

(22) Date de dépôt: **26.12.90**

Le dossier contient des informations techniques présentées postérieurement au dépôt de la demande et ne figurant pas dans le présent fascicule.

# (54) Composés non ioniques iodés, leur procédé de préparation et produits de contraste en contenant.

(30) Priorité: **05.01.90 FR 9000106**

(43) Date de publication de la demande:
**17.07.91 Bulletin 91/29**

(45) Mention de la délivrance du brevet:
**30.11.94 Bulletin 94/48**

(84) Etats contractants désignés:
**AT BE CH DE DK ES FR GB GR IT LI LU NL SE**

(56) Documents cités:
**EP-A- 0 015 867**
**EP-A- 0 357 467**
**DE-A- 3 429 949**

**Investigative Radiology, vol.15,323-328,(1980)**

(73) Titulaire: **GUERBET S.A.**
**15, rue des Vanesses**
**Z.A.C. Paris Nord II**
**F-93420 Villepinte (FR)**

(72) Inventeur: **Schaefer, Michel**
**4 Rue François Girardon**
**F-91380 Chilly-Mazarin (FR)**
Inventeur: **Dugast-Zrihen, Maryse**
**107 Rue Bobillot**
**F-75013 Paris (FR)**
Inventeur: **Guillemot, Michel**
**182 bis, Boulevard Péreire**
**F-75017 Paris (FR)**
Inventeur: **Doucet, Didier**
**13 Rue Amédée Dunois**
**F-93190 Livry-Gargan (FR)**
Inventeur: **Meyer, Dominique**
**6 Rue de Metz**
**F-94100 Saint-Maur (FR)**

(74) Mandataire: **Le Guen, Gérard et al**
**CABINET LAVOIX**
**2, place d'Estienne d'Orves**
**F-75441 Paris Cédex 09 (FR)**

**EP 0 437 144 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne des composés utilisables dans des produits de contraste pour la radiographie.

On utilise depuis longtemps comme produits de contraste des composés iodobenzéniques présentant sur le noyau benzénique plusieurs atomes d'iode, en général 3 atomes d'iode par noyau benzénique, et divers autres substituants. Ces autres substituants sont des groupes pharmacologiquement acceptables qui permettent l'administration des composés chez l'homme et les animaux. Ces substituants sont d'une manière générale choisis, d'une part, pour conférer aux composés une hydrosolubilité suffisante en vue de l'administration de ces composés en solution aqueuse et, d'autre part, pour conférer à ces composés une tolérance suffisante par l'organisme humain.

A cet effet, on a proposé des structures non-ioniques, c'est-à-dire des dérivés iodobenzéniques possédant des substituants non-ioniques.

Ainsi, dans le brevet FR-A-2 053 037, on a proposé des composés carbamoyl iodobenzéniques contenant au total au moins un groupe N-hydroxy alcoyle et au moins deux groupes hydroxy.

Un composé illustrant cette classe est le métrizamide qui s'avère être toutefois d'une stabilité limitée.

Dans la demande de brevet européen 89 401 509.8 on a proposé des composés non-ioniques qui sont bien tolérés par l'organisme humain et qui présentent une bonne stabilité en solution aqueuse.

Ces composés répondent à la formule :

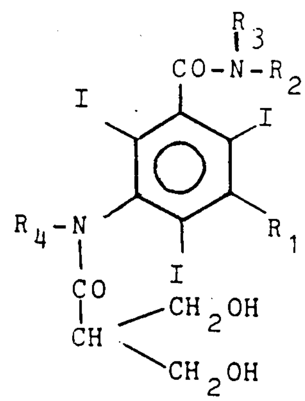

dans laquelle
$R_1$ représente un groupe de formule

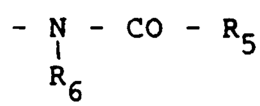

$R_5$ représentant un groupe alkyle en $C_1$-$C_4$, un groupe hydroxy alkyle en $C_1$-$C_4$ ou un groupe polyhydroxyalkyle en $C_1$-$C_4$,
$R_6$ représentant un atome d'hydrogène, un groupe alkyle en $C_1$-$C_4$, un groupe hydroxyalkyle en $C_1$-$C_4$ ou un groupe polyhydroxyalkyle en $C_1$-$C_4$,
ou un groupe de formule

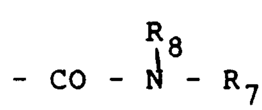

$R_7$ représentant un groupe hydroxyalkyle en $C_1$-$C_4$ ou un groupe polyhydroxyalkyle en $C_1$-$C_4$
$R_8$ représentant un atome d'hydrogène ou un groupe alkyle en $C_1$-$C_4$
$R_2$ représente un atome d'hydrogène, un groupe hydroxyalkyle en $C_1$-$C_4$ ou un groupe polyhydroxyalkyle en $C_1$-$C_4$,
$R_3$ représente un atome d'hydrogène ou un groupe alkyle en $C_1$-$C_4$, et

$R_4$ représente un atome d'hydrogène, un groupe alkyle en $C_1$-$C_4$, un groupe hydroxyalkyle en $C_1$-$C_4$ ou un groupe polyhydroxyalkyle en $C_1$-$C_4$.

La présente invention vise à fournir de nouveaux composés appartenant à la famille des composés de formule I, qui se caractérisent à la fois par une bonne stabilité et par une bonne solubilité en milieu aqueux.

Les composés selon l'invention répondent à la formule générale suivante :

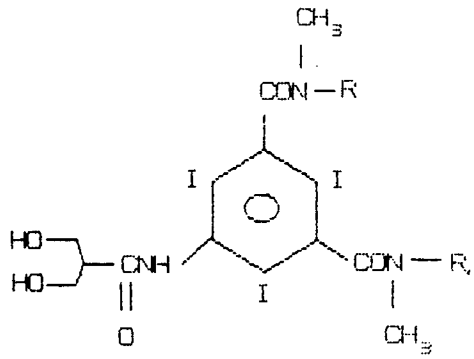

(I)

dans laquelle R représente le groupe 2,3-dihydroxypropyle ou le groupe 1,3-dihydroxy-2-propyle.

L'un des composés selon l'invention est le 5-[3-hydroxy-2-(hydroxyméthyl)-propionamido]-N,N'-diméthyl-N,N'-bis-(2,3-dihydroxypropyl)-2,4,6-triiodoisophtalamide, c'est-à-dire le composé de formule

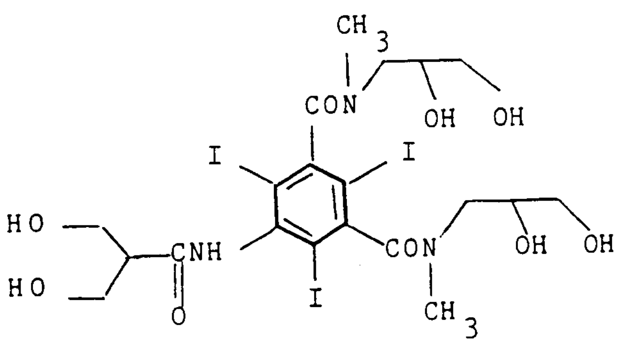

(A)

L'autre composé selon l'invention est le 5-[3-hydroxy-2-(hydroxymethyl-propionamido]-N,N'-dimethyl-N,N'-bis-(1,3-dihydroxy-2-propyl)-2,4,6-triiodoisophtalamide répondant à la formule ci-après :

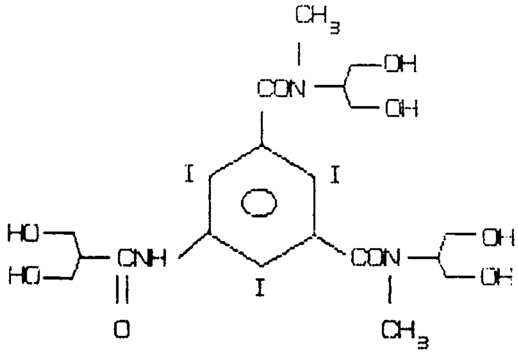

(B)

Ces formules couvrent non seulement les racémiques mais également l'ensemble des stéréoisomères associés à la présence de carbone asymétrique et à l'empêchement de rotation de certaines liaisons dû à l'encombrement stérique apporté par les atomes d'iode.

De manière inattendue, le composé de formule A se caractérise par une meilleure solubilité en milieu aqueux que le composé de l'exemple 1 de la demande de brevet européen citée ci-dessus, à savoir le 5-[3-hydroxy-2-(hydroxyméthyl)-N-(2,3-dihydroxypropyl)propionamido]-N'-N''-bis-(2-hydroxy-éthyl)-2,4,6-triiodo-isophtalamide.

De plus, les composés selon l'invention présentent, par rapport au composé de l'exemple 1 de la demande de brevet citée ci-dessus, l'avantage d'être plus facilement accessible dans la mesure où ils ne nécessitent pas de stade de N-alkylation finale qui pose des problèmes de purification au niveau industriel.

Les composés selon l'invention peuvent être obtenus selon un procédé consistant à :

a) faire réagir un dichlorure d'acide de formule :

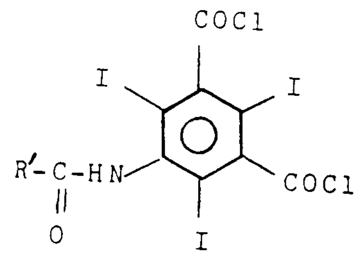

(II)

R' désignant un groupe $-CH-(CH_2OH)_2$ dont les groupes hydroxy sont protégés,

avec une amine choisie parmi le l-(N-méthylamino) propane-2,3-diol et le 2-(N-méthylamino) propane-1,3-diol, de façon à obtenir un composé de formule

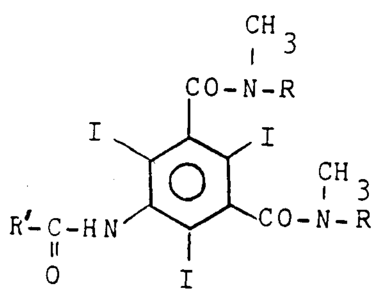

(III)

et

b) éliminer les groupes protecteurs du groupe R'.

Les composés de formule II sont décrits dans FR-A-2 632 304.

La présente invention a également pour objet des produits de contraste qui comprennent au moins les composés selon l'invention.

Ces produits de contraste sont utilisés chez l'homme et les animaux à des fins radiologiques.

La forme pharmaceutique préféré des produits de contraste selon l'invention est constituée par des solutions aqueuses du composé.

Les solutions aqueuses contiennent généralement un total de 5 à 100 g de composé selon l'invention tion pour 100 ml et la quantité injectable de telles solutions peut varier généralement de 1 à 1000 ml.

Les solutions aqueuses des composés selon l'invention peuvent également contenir certains additifs comme :

- du chlorure de sodium à des concentrations comprises entre 0,1 à 10 mM
- de l'EDTA disodique à des concentrations comprises entre 0,1 et 2 mM
- du citrate de sodium à des concentrations comprises entre 0,1 et 10 mM
- de l'héparine à des doses comprises entre 10 et 100 unités pour 100 ml de solution.

Ces compositions peuvent être administrées par toutes les voies classiquement utilisées pour les produits de contraste non ioniques iodés. Ainsi elles peuvent être administrées par voie entérale ou parentérale (voie intraveineuse, intraartérielle, opacification des cavités) et en particulier dans l'espace sous-arachnoidien.

On donnera ci-après un exemple de composition selon la présente invention.

| | |
|---|---|
| Composition | |
| Composé A selon l'invention | 65 g |
| Eau pour préparation injectable | |
| QSP | 100 ml |

Les exemples suivant illustrent la préparation des composés selon l'invention.

EXEMPLE 1

Préparation du 5-[3-hydroxy-2-(hydroxy méthyl)-propionamido]-N-N'-diméthyl-N,N'-bis-(2,3-dihy droxypropyl)-2,4,6-triiodoisophtalamide.

a) Préparation du [2-isopropyl-1,3-dioxanne-5-carboxamido]-N,N'-diméthyl-N,N'-bis-(2,3-dihydroxypropyl)-2,4,6-triiodoisophtalamide.

74 g (98 mmoles) de dichlorure de 5-[2-iso-propyl-1,3-dioxanne-5-carboxamido]-2,4,6-triiodoisophtaloyle sont mis en suspension dans 300 ml d'isopropanol contenant 41 ml (294 mmoles) de triéthylamine. On ajoute goutte à goutte 31 g (295 mmoles) de N-méthyl-aminopropane-2,3-diol. L'agitation est maintenue 12 h à température ambiante. Le chlorhydrate de triéthylamine est éliminé par filtration.

Le filtrat est évaporé à sec, repris à l'eau et élué sur résine $OH^-$ IRA 67.

Après évaporation, le produit est purifié par passage sur silice silanisée (Kieselgel 60 Merck) avec l'eau comme éluant.

Après évaporation à sec, on recueille 60 g de poudre blanche avec un rendement de 68,5 %.

| | | | | |
|---|---|---|---|---|
| Dosage d'iode | : 96,4 % | | | |
| Pureté HPLC | : 97 % | Hypersil CB 25 cm 5 µm | | |
| | | $NaH_2PO_4$ | 0,01 M | 60 |
| | | Méthanol | | 40 |
| CCM $SiO_2$, Rf | 0,12 | | | |
| | 0,25 | | | |
| | 0,30 | 0,36 | | |

Eluant $CHCl_3$ 55, MeOH 30, $NH_3H_2O$ 10

RMN (DMSO) $^1H$ 200 MHz

0,9 ppm (doublet) $CH_3$ (6H) ; 2,8 ppm (singulet) N-$CH_3$ (3H); 3 ppm (singulet) N-$CH_3$(3H); 3,3 ppm (multiplet) N-$CH_2$ et CH (5H) ; 3,5-3,9 ppm (massif) $CH_2$ et CH (8H); 4,3 ppm (quadruplet) CH (3H); 4,6 ppm (triplet) OH (2H); 4,7 ppm (doublet) OH (2H); 10,2 ppm (massif élargi) NH (1H).

b) Préparation du 5-[3-hydroxy-2-(hydroxyméthyl)-propionamido]-N,N'-diméthyl-N,N'-bis-(2,3-dihy droxypropyl)-2,4,6-triiodoisophtalamide.

45 g (50,6 mmole) du composé décrit en a) sont dissous dans 101 ml (10 eq) d'acide chlorhydrique 5 N. L'agitation est maintenue 12 h à température ambiante. La solution est filtrée et évaporée à sec. Le solide obtenu est repris dans 100 $cm^3$ d'éther éthylique puis filtré et élué sur silice silanisé (Kieselgel 60 Merck) avec de l'eau. On recueille, après évaporation à sec, 38 g de poudre blanche.

Rendement = 90 %

Dosage d'iode = 98,3 %

Pureté HPLC ⩾ 98 % Hypersil C8 25 cm 5 µ

| | | |
|---|---|---|
| $NaH_2PO_4$ | 0,01 M | 95 |
| MeOH | | 5 |

CCM $SiO_2$ RF 0,56
0,63
0,67

Eluant $CHCl_3$ 55, MeOH 30, $NH_3$,$H_2O$ 10

RMN (DMSO) $^1H$ 200 MHz

2,7 ppm (massif) CH (1H); 2,85 ppm (singulet élargi) N-$CH_3$ (3H); 3,08 ppm (doublet mal résolu) N-$CH_3$ - (3H); 3,10-3,35 ppm (multiplet) N-$CH_2$; 3,45 ppm (quadruplet) $CH_2$ (4H); 3,6-4 ppm (massif) OH (6H); 9,9 ppm (massif) NH (1H).

EXEMPLE 2

Préparation du 5-[3-hydroxy-2-(hydroxyméthyl -propionamido]-N,N'-diméthyl-N,N'-bis-(1,3-dihydroxy-2 -propyl)-2,4,6-triiodoisophtalamide)

a) Préparation du (2-isopropyl-1,3-dioxanne-6-carboxamido)-N,N'-diméthyl-N,N'-bis-(1,3-dihydroxy-2 -propyl)-2,4,6-triiodoisophtalamide.

1,46 g (13,9 mmole) de N-méthyl-aminopropane -1,3-diol (synthétisée selon la demande de brevet européen EP-A-025 083 déposée par EPROVA A.G.) est dissous dans un mélange composé de 20 $cm^3$ de N-N-diméthylacétamide et 2 $cm^3$ (14,4 mmole) de triéthylamine. On ajoute par portion 3,5 g (4,65 mmole) de dichlorure de 5-(2-isopropyl-1,3-dioxanne-5-carboxamido)-2,4,6-triiodoisophtaloyle. L'agitation est maintenue 3 heures à 30° C, puis 12 heures à température ambiante. Le chlorhydrate de triéthylamine est éliminé par filtration.

Le filtrat est évaporé à sec, et le résidu est cristallisé dans un mélange composé de 20 $cm^3$ d'isopropanol et 200 $cm^3$ d'éther éthylique.

Après filtration et séchage, on recueille 3,6 g de poudre blanche avec un rendement de 87 %.

CCM $SiO_2$ Rf 0,16
0,28

Eluant toluène 60 - méthyl-éthylcétone 35 - acide formique 25.

RMN (DMSO) $^1H$ : 200 MHz.

0,9 ppm (doublet) $CH_3$ (6H) ; 1,7 ppm (massif) CH (1H); 2,7 ppm (singulet) N-$CH_3$ (3H) isomère Z; 2,99 ppm (singulet) N-$CH_3$ (3H) isomère E ; 3,4-4 ppm (multiplet) CH, $CH_2$ (15 H); 4,8 ppm (massif) $CH_2OH$ (4H) ; 10,2 ppm (massif) NH (1H).

b) Préparation du 5-[3-hydroxy-2-(hydroxyméthyl-propionamido]-N,N'-diméthyl-N,N'-bis(1,3-dihydroxy-2-propyl)-2,4,6-triiodoisophtalamide.

3 g (3,4 mmole) du composé décrit en a) sont dissous dans 10 $cm^3$ (15 eq) d'acide chlorhydrique 5N. L'agitation est maintenue 3 heures à 45° C, puis 12 heures à température ambiante. La solution est évaporée à sec. Le résidu obtenu est repris par 50 $cm^3$ d'éther éthylique, filtré, puis est dissous dans 50 $cm^3$ d'eau, avant d'être élué sur résine $H^+$ (IRN 77) et $OH^-$ (IRA 68).

On recueille après évaporation à sec 1,7 g de poudre blanche avec un rendement de 60,7 %.

Pureté en iode : 98,4 %

<u>CCM</u> $SiO_2$ Rf 0,26

0,33

0,45

Eluant butanol 50, eau 25, acide acétique 11.

<u>RMN</u> (DMSO)$^1$H 200 MHz.

2,7 ppm (singulet élargi) N-$CH_3$ (3H), isomère Z ; 3 ppm (singulet élargi- N-$CH_3$ (3H), isomère E ; 3,3-4,05 ppm (massif mal résolu) CH, C<u>H</u>$_2$OH (15 H); 4,45 ppm (massif mal résolu) $CH_2$O<u>H</u> (6 H); 9,8 ppm (massif) NH (1H).

<u>RMN</u> (DMSO)[13] C 200 MHz.

170,6 ppm (2 $\underset{\overset{\|}{O}}{C}$); 172 ppm (1 $\underset{\overset{\|}{O}}{C}$); 149 ppm ($C_{Ar}$-$\underset{\overset{\|}{O}}{C}$) ;

145 ppm ($C_{Al}$-NH); 100-98-90 ppm ($C_{Ar}$-I); 61-57-52 ppm (C-H); 59,1-59,9 ppm ($CH_2$-OH); 32,8-30,1 ppm ($CH_3$-N).

## Revendications

**Revendications pour les Etats contractants suivants : AT, BE, CH, DE, DK, FR, GB, GR, IT, LI, LU, NL, SE**

**1.** Composés répondant à la formule générale:

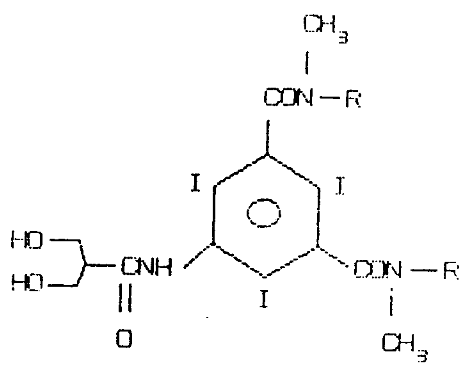

dans laquelle R est choisi parmi les groupes 2,3-dihydroxypropyle et 1,3-dihydroxy-2-propyle.

**2.** Le 5-[3-hydroxy-2-(hydroxyméthyl)- propionamido]-N,N'-diméthyl-N,N'bis(2,3-dihydroxypropyl)-2,4,6-triiodoisophtalamide.

**3.** Le 5-[3-hydroxy-2-(hydroxyméthyl)-propionamido]-N,N'-diméthyl-N,N'bis(1,3-dihydroxy-2-propyl)-2,4,6-triiodoisophtalamide.

**4.** Procédé de préparation d'un composé selon la revendication 1, consistant à

a) faire réagir un dichlorure d'acide de formule :

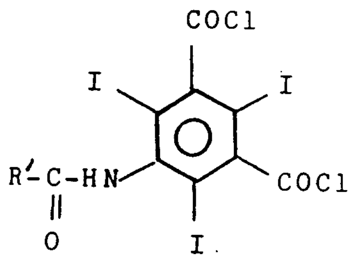

(II)

R' désignant un groupe $-CH-(CH_2OH)_2$ dont les groupes hydroxy sont protégés, avec une amine choisie parmi le 1(N-méthylamino) propane-2,3-diol et le 2-(N-méthylamino) propane-1,3-diol, de façon à obtenir un composé de formule

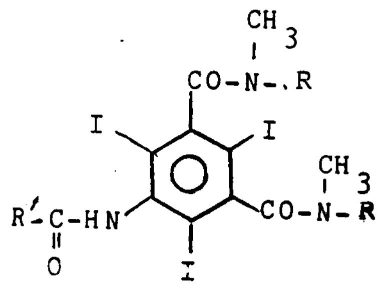

(III)

et

b) éliminer les groupes protecteurs du groupe R'.

**5.** Produit de contraste, caractérisé en ce qu'il contient au moins un composé selon la revendication 1.

**6.** Produit de contraste, caractérisé en ce qu'il contient au moins le composé de la revendication 2.

**7.** Produit de contraste, caractérisé en ce qu'il contient au moins le composé de la revendication 3.

**8.** Produit de contraste selon la revendication 6, caractérisé en ce qu'il est constitué par une solution aqueuse du composé.

**9.** Produit de contraste selon la revendication 7, caractérisé en ce qu'il est constitué par une solution aqueuse du composé.

**Revendications pour l'Etat contractant suivant : ES**

**1.** Procédé de préparation d'un composé répondant à la formule générale:

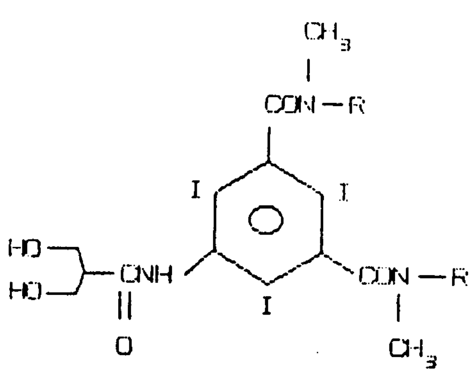

(I)

dans laquelle R est choisi parmi les groupes 2,3-dihydroxypropyle et 1,3-dihydroxy-2-propyle, consistant à

a) faire réagir un dichlorure d'acide de formule :

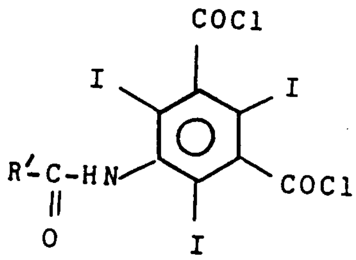

(II)

R' désignant un groupe $-CH-(CH_2OH)_2$ dont les groupes hydroxy sont protégés, avec une amine choisie parmi le 1(N-méthylamino) propane-2,3-diol et le 2-(N-méthylamino) propane-1,3-diol, de façon à obtenir un composé de formule

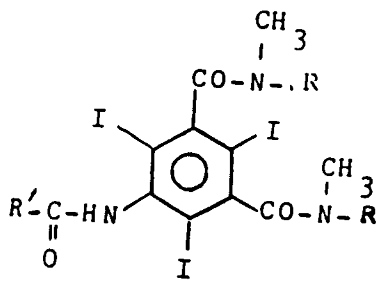

(III)

et

b) éliminer les groupes protecteurs du groupe R'.

2. Procédé de préparation d'un produit de contraste, caractérisé en ce que l'on met un composé de formule I telle que définie à la revendication 1 sous une forme administrable.

3. Procédé selon la revendication 2, caractérisé en ce que l'on met le composé sous forme d'une solution aqueuse.

## Claims

**Claims for the following Contracting States : AT, BE, CH, DE, DK, FR, GB, GR, IT, LI, LI, NL, SE**

1. Compounds corresponding to the general formula:

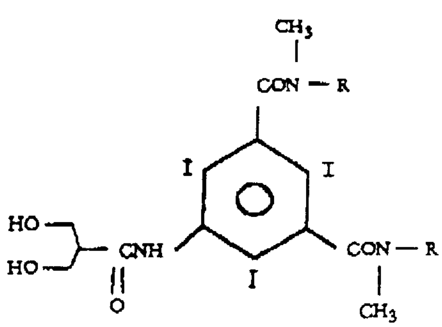

in which R is chosen from 2,3-dihydroxypropyl and 1,3-dihydroxy-2-propyl groups.

**2.** 5-[3-Hydroxy-2-(hydroxymethyl)propionamido]-N,N'-dimethyl-N,N'-bis(2,3-dihydroxypropyl)-2,4,6-triiodoisophthalamide.

**3.** 5-[3-Hydroxy-2-(hydroxymethyl)propionamido]-N,N'-dimethyl-N,N'-bis(1,3-dihydroxy-2-propyl)-2,4,6-triiodoisophthalamide.

**4.** Process for the preparation of a compound according to Claim 1, consisting in

a) reacting a diacyl chloride of formula:

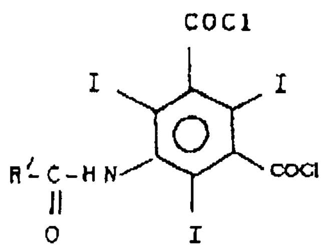

(II)

R' denoting a $-CH-(CH_2OH)_2$ group in which the hydroxyl groups are protected, with an amino chosen from 1-(N-methylamino)-propane-2,3-diol and 2-(N-methylamino)-propane-1,3-diol, so as to obtain a compound of formula

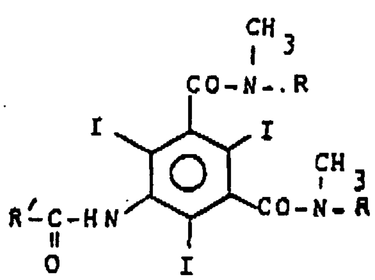

(III)

and

b) removing the protecting groups of the group R'.

**5.** Contrast medium, characterized in that it contains at least one compound according to Claim 1.

**6.** Contrast medium, characterized in that it contains at least the compound of Claim 2.

**7.** Contrast medium, characterized in that it contains at least the compound of Claim 3.

**8.** Contrast medium according to Claim 6, characterized in that it consists of an aqueous solution of the compound.

**9.** Contrast medium according to Claim 7, characterized in that it consists of an aqueous solution of the compound.

**Claims for the following Contracting State : ES**

**1.** Process for the preparation of a compound corresponding to the general formula:

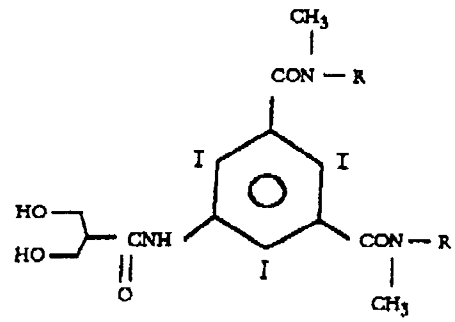

in which R is chosen from 2,3-dihyroxypropyl and 1,3-dihydroxy-2-propyl groups, consisting in

a) reacting a diacyl chloride of formula:

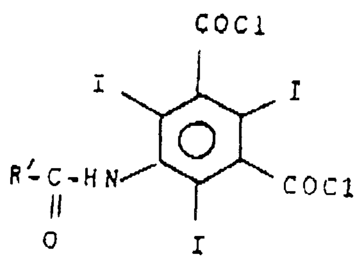

(II)

R' denoting a $-CH-(CH_2OH)_2$ group in which the hydroxyl groups are protected,
with an amine chosen from 1-(N-methylamino)-propane-2,3-diol and 2-(N-methylamino)-propane-1,3-diol, so as to obtain a compound of formula

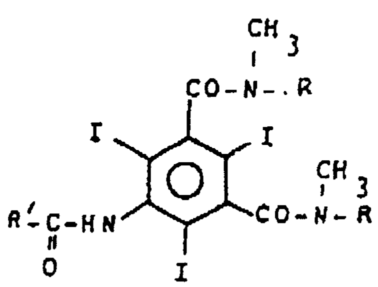

(III)

and

b) removing the protecting groups of the group R'.

**2.** Process for the preparation of a contrast medium, characterized in that a compound of formula I as defined in Claim 1 is placed in a form which may be administered.

**3.** Process according to Claim 2, characterized in that the compound is placed in the form of an aqueous solution.

**Patentansprüche**

**Patentansprüche für folgende Vertragsstaaten : AT, BE, CH, DE, DK, FR, GB, GR, IT, LI, LU, NL, SE**

1. Verbindungen der allgemeinen Formel:

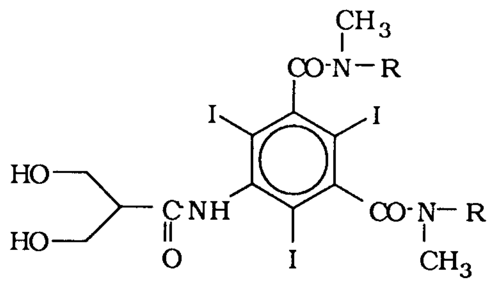

(I)

in der R aus 2,3-Dihydroxypropyl- und 1,3-Dihydroxy-2-propyl-gruppen ausgewählt ist.

2. 5-[3-Hydroxy-2-(hydroxymethyl)-propionamido]-N,N'-dimethyl-N,N'-bis(2,3-dihydroxypropyl)-2,4,6-triiodisophthalamid.

3. 5-[3-Hydroxy-2-(hydroxymethyl)-propionamido]-N,N'-dimethyl-N,N'-bis(1,3-dihydroxy-2-propyl)-2,4,6-triiodisophthalamid.

4. Verfahren zur Herstellung einer Verbindung nach Anspruch 1, **dadurch gekennzeichnet**, daß man
   a) ein Disäurechlorid der Formel:

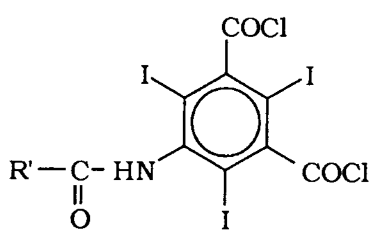

(II)

in der R' eine Gruppe der Formel $-CH-(CH_2OH)_2$, deren Hydroxylgruppen geschützt sind, bedeutet, mit einem aus 1-(N-Methylamino)-propan-2,3-diol und 2-(N-Methylamino)-propan-1,3-diol ausgewählten Amin in der Weise umsetzt, daß man eine Verbindung der Formel:

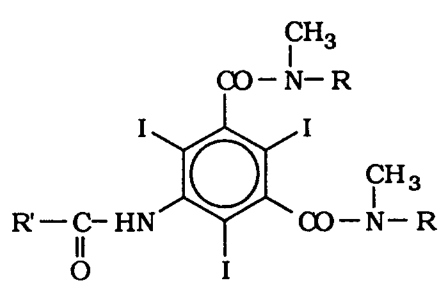

(III)

erhält und
   b) die Schutzgruppen der Gruppe R' entfernt.

5. Kontrastmittel, **dadurch gekennzeichnet**, daß es mindestens eine Verbindung nach Anspruch 1 enthält.

6. Kontrastmittel, **dadurch gekennzeichnet**, daß es mindestens die Verbindung nach Anspruch 2 enthält.

7. Kontrastmittel, **dadurch gekennzeichnet**, daß es mindestens die Verbindung nach Anspruch 3 enthält.

8. Kontrastmittel nach Anspruch 6, **dadurch gekennzeichnet**, daß es eine wäßrige Lösung der Verbindung umfaßt.

9. Kontrastmittel nach Anspruch 7, **dadurch gekennzeichnet**, daß es eine wäßrige Lösung der Verbindung umfaßt.

**Patentansprüche für folgenden Vertragsstaat : ES**

1. Verfahren zur Herstellung einer Verbindung der allgemeinen Formel:

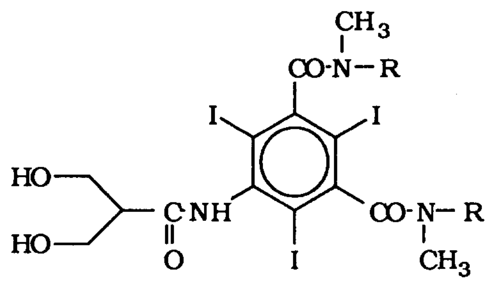

(I)

in der R aus 2,3-Dihydroxypropyl- und 1,3-Dihydroxy-2-propyl-gruppen ausgewählt ist, **dadurch gekennzeichnet**, daß man

a) ein Disäurechlorid der Formel:

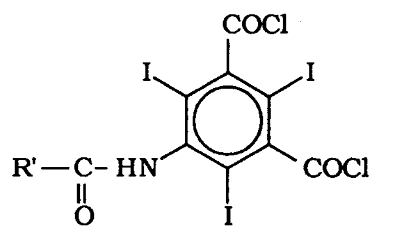

(II)

in der R' eine Gruppe der Formel -CH-($CH_2OH)_2$, deren Hydroxylgruppen geschützt sind, bedeutet, mit einem aus 1-(N-Methylamino)-propan-2,3-diol und 2-(N-Methylamino)-propan-1,3-diol ausgewählten Amin in der Weise umsetzt, daß man eine Verbindung der Formel:

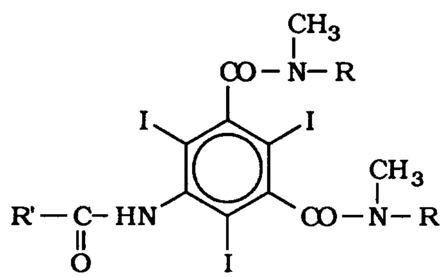

(III)

erhält und

b) die Schutzgruppen der Gruppe R' entfernt.

2. Verfahren zur Herstellung eines Kontrastmittels, **dadurch gekennzeichnet**, daß man eine Verbindung der Formel I, wie sie in Anspruch 1 definiert ist, in eine verabreichbare Form bringt.

3. Verfahren nach Anspruch 2, **dadurch gekennzeichnet**, daß man die Verbindung in die Form einer wäßrigen Lösung bringt.